# EUROPEAN PATENT SPECIFICATION

(11) **EP 2 958 552 B1**
(45) Date of publication and mention of the grant of the patent: **21.06.2017**
(21) Application number: 14705162.7
(22) Date of filing: 18.02.2014
(51) Int. Cl.: A61K 9/16, A61K 9/20, A61K 9/28

(54) **PHARMACEUTICAL COMPOSITION COMPRISING N-[3-CHLORO-4-(3-FLUOROBENZYLOXY)PHENYL]-6-[5({[2-(METHYLSULFONYL)ETHYL]AMINO}METHYL)-2-FURYL]QUINAZOLIN-4-AMINE OR A PHARMACEUTICALLY ACCEPTABLE SALT, SOLVATE OR SOLVATED SALT THEREOF**
PHARMAZEUTISCHE ZUSAMMENSETZUNG MIT N-[3-CHLOR-4-(3-FLUORBENZYLOXY)PHENYL]-6-[5({[2-(METHYLSULFONYL)ETHYL]AMINO}METHYL)-2-FURYL]CHINAZOLIN-4-AMIN ODER EIN PHARMAZEUTISCH AKZEPTABLES SALZ, SOLVAT ODER SOLVATISIERTES SALZ DARAUS
COMPOSITION PHARMACEUTIQUE COMPRENANT N-[3-CHLORO-4-(3-FLUOROBENZYLOXY)PHENYL]-6-[5({[2-(METHYLSULFONYL)ETHYL]AMINO}METHYL)-2-FURYL]QUINAZOLIN-4-AMINE OU UN SEL PHARMACEUTIQUEMENT ACCEPTABLE, UN SOLVATE OU SEL SOLVATÉ DE CELUI-CI

(30) Priority: 19.02.2013 EP 13155741
(43) Date of publication of application: 30.12.2015
(73) Proprietor: Hexal AG, 83607 Holzkirchen (DE)
(72) Inventor: ROTHER, Patrick, 23858 Reinfeld (DE)
(74) Representative: Global IP Europe Patentanwaltskanzlei
(86) International application number: PCT/EP2014/053087
(87) International publication number: WO 2014/128107

(56) References cited:
- EP-A1- 2 158 912
- WO-A1-2006/113649
- WO-A1-2010/099150

## Description

### FIELD OF THE INVENTION

The present invention relates to a pharmaceutical composition comprising N-[3-chloro-4-(3-fluorobenzyloxy)phenyl]-6-[5({[2-(methylsulfonyl)ethyl]amino}methyl)-2-furyl]quinazolin-4-amine or a pharmaceutically acceptable salt, solvate or solvated salt thereof, and a process for preparing the pharmaceutical composition.

### BACKGROUND OF THE INVENTION

N-[3-chloro-4-(3-fluorobenzyloxy)phenyl]-6-[5({[2-(methylsulfonyl)ethyl]amino}methyl)-2-furyl]quinazolin-4-amine is hereinafter referred to as lapatinib in accordance with the international non-proprietary name (INN) given by the World Health Organization (WHO). Lapatinib ditosylate monohydrate was shown to act as an inhibitor of the intracellular tyrosine kinase domains of certain epidermal growth factor receptors, such as ErbB-1 and ErbB-2, and is currently used in the treatment of patients with breast cancer, whose tumours overexpress ErbB-2.

WO 2006/113649 discloses oral pharmaceutical compositions comprising an active ingredient, which is for example lapatinib or a salt or solvate thereof, together with a binder, and optionally a disintegrant and a lubricant.

WO 2010/023187 discloses pharmaceutical compositions comprising lapatinib or a pharmaceutically acceptable salt thereof as active pharmaceutical ingredient, wherein the active pharmaceutical ingredient is present in an amount of more than 60% by weight based on the total weight of the composition.

WO 2010/023188 discloses pharmaceutical compositions comprising lapatinib or a pharmaceutically acceptable salt thereof wherein a unit dose of the composition contains 1200 to 1300 mg of the active pharmaceutical ingredient calculated as the free base.

### SUMMARY OF THE INVENTION

It has now been found that a pharmaceutical composition comprising lapatinib or a pharmaceutically acceptable salt or solvate or solvated salt thereof, at least one binder, at least one disintegrant, at least one lubricant, and at least one filler, wherein the filler comprises microcrystalline cellulose in an amount of 10 to 30% by weight, relative to the weight of the pharmaceutical composition, is associated with advantages compared to prior art compositions. In particular, the pharmaceutical composition according to the present invention can be prepared in a cost and time efficient way, especially large scale manufacturing can be done at high speed without producing a significant number of defective products with regard to tablet weight and tablet appearance, whereby the pharmaceutical composition shows a desired dissolution behaviour.

### DETAILED DESCRIPTION OF THE INVENTION

Lapatinib and the pharmaceutically acceptable salts, solvates and solvated salts thereof can be prepared as described in WO 99/35146 and WO 02/02552.

Lapatinib or the pharmaceutically acceptable salt or solvate or solvated salt thereof is preferably present in the pharmaceutical composition according to the present invention in an amount of from 30 to 60% by weight, relative to the weight of the pharmaceutical composition, more preferably in an amount of from 40 to 60% by weight, relative to the weight of the pharmaceutical composition.

Preferably, the pharmaceutical composition according to the present invention contains 250 mg, 500 mg, 750 mg, 1000 mg or 1250 mg of lapatinib or the pharmaceutically acceptable salt or solvate or solvated salt thereof (calculated as the free base). Most preferably, the pharmaceutical composition according to the present invention contains 250 mg of lapatinib or the pharmaceutically acceptable salt or solvate or solvated salt thereof (calculated as the free base).

Pharmaceutically acceptable salts of lapatinib include, for example, the hydrochloride, hydrobromide, sulfate, nitrate, acetate, tartrate, citrate, fumarate, lactate, malate, maleate, mesylate, pamoate, oxalate, gluconate, salicylate, benzoate, succinate, and tosylate salts.

Preferred pharmaceutically acceptable salts of lapatinib include the monomesylate, the dimesylate, the monotosylate and the ditosylate salt, in particular the ditosylate anhydrate salt. The pharmaceutically acceptable solvates of lapatinib include the hydrates of lapatinib. The monohydrate of lapatinib is particularly preferred. Pharmaceutically acceptable solvated salts of lapatinib include lapatinib mesylate monohydrate, lapatinib dimesylate monohydrate, lapatinib tosylate monohydrate, and lapatinib ditosylate monohydrate. Lapatinib ditosylate monohydrate is most preferred.

In a preferred embodiment, the pharmaceutical composition of the present invention comprises a binder selected from the group consisting of gelatine, polyvinyl pyrrolidone (PVP), polyvinyl acetate, polyvinyl alcohol, hydroxypropylcellulose (HPC), hydroxypropylmethylcellulose (HPMC) and combinations thereof.

The binder is preferably present in the pharmaceutical composition of the present invention in an amount of from 1 to 20% by weight, relative to the weight of the pharmaceutical composition, preferably in an amount of from 5 to 10% by weight, relative to the weight of the pharmaceutical composition.

In a further preferred embodiment, the pharmaceutical composition of the present invention comprises a disintegrant selected from the group consisting of sodium starch glycolate, croscarmellose sodium, crospovidone and combinations thereof.

Crospovidone is a cross-linked polyvinylpyrrolidone. It is formed by so-called popcorn-polymerization of vinylpyrrolidone. Examples of crospovidone include Kollidon CL, Kollidon CL-M, PolyKoVidone, Polyplasdone XL and Polyplasdone XL-10. A preferred example is Polyplasdone XL.

The disintegrant is preferably present in the pharmaceutical composition of the present invention in an amount of from 0.5 to 8% by weight, relative to the weight of the pharmaceutical composition, preferably in an amount of from 4 to 6% by weight, relative to the weight of the pharmaceutical composition.

In a further preferred embodiment, the pharmaceutical composition of the present invention comprises a lubricant selected from the group consisting of magnesium stearate, calcium stearate, talc, aluminum monostearate, aluminum distearate, aluminum tristearate, zinc stearate, stearic acid, sodium stearyl fumarate, magnesium behenate, calcium behenate, magnesium arachinate, calcium arachinate, and combinations thereof.

The lubricant is preferably present in the pharmaceutical composition of the present invention in an amount of from of 0.01 to 3% by weight, relative to the weight of the pharmaceutical composition, preferably in an amount of from 0.5 to 2% by weight, relative to the weight of the pharmaceutical composition.

The microcrystalline cellulose is present in the pharmaceutical composition of the present invention in an amount of 10 to 30% by weight, relative to the weight of the pharmaceutical composition. Preferably, the microcrystalline cellulose has a median particle size of 50 to 100 µm. The particle size distribution is measured by laser diffraction on a Mastersizer 2000 with dry powder dispersion unit Scirocco 2000 (Measurement conditions: Sample weight is 1000 mg; the feed rate with a micro feed tray is 80%; the dispersion pressure is 2 bar; the diffraction method is Frauenhofer with analysis model "general purpose" and refraction index of particles is 0.000; measurement time is 12 s; background time is 12 s).

In a preferred embodiment, the pharmaceutical composition of the present invention comprises mannitol, more preferably in an amount of 10 to 20% by weight, relative to the weight of the pharmaceutical composition.

In a further preferred embodiment, the pharmaceutical composition of the present invention comprises calcium hydrogen phosphate. The calcium hydrogen phosphate may be present in the form of calcium hydrogen phosphate anhydrate or calcium hydrogen phosphate dihydrate. In a particularly preferred embodiment, the pharmaceutical composition of the present invention comprises calcium hydrogen phosphate in an amount of 10 to 20% by weight, relative to the weight of the pharmaceutical composition.

In a further preferred embodiment, the pharmaceutical composition of the present invention comprises lactose. In a particularly preferred embodiment, the pharmaceutical composition of the present invention comprises lactose in an amount of 10 to 20% by weight, relative to the weight of the pharmaceutical composition. The lactose can be present as alpha-lactose anhydrate, alpha-lactose monohydrate, beta-lactose, amorphous lactose, crystalline lactose or mixtures thereof. Alpha-lactose monohydrate is most preferred.

In a further preferred embodiment, the pharmaceutical composition of the present invention comprises starch. The starch is, for example, maize starch, wheat starch, rice starch and/or potato starch, and can be present as natural starch and/or pre-treated starch, such as pregelatinized starch or thermally treated starch. In a particularly preferred embodiment, the pharmaceutical composition of the present invention comprises the starch in an amount of 10 to 20% by weight, relative to the weight of the pharmaceutical composition.

In a further preferred embodiment, the pharmaceutical composition of the present invention comprises
lapatinib or a pharmaceutically acceptable salt or solvate or solvated salt thereof,
at least one binder selected from the group consisting of gelatine, polyvinyl pyrrolidone, polyvinyl acetate, polyvinyl alcohol, hydroxypropylcellulose, hydroxypropylmethylcellulose and combinations thereof;
at least one disintegrant selected from the group consisting of sodium starch glycolate, croscarmellose sodium, crospovidone and combinations thereof;
at least one lubricant selected from the group consisting of magnesium stearate, calcium stearate, talc, aluminum monostearate, aluminum distearate, aluminum tristearate, zinc stearate, stearic acid, sodium stearyl fumarate, magnesium behenate, calcium behenate, magnesium arachinate, calcium arachinate, and combinations thereof;
at least one filler which comprises microcrystalline cellulose in an amount of 10 to 30% by weight, relative to the weight of the pharmaceutical composition.

In a further preferred embodiment, the pharmaceutical composition of the present invention comprises
lapatinib or a pharmaceutically acceptable salt or solvate or solvated salt thereof,
at least one binder selected from the group consisting of gelatine, polyvinyl pyrrolidone, polyvinyl acetate, polyvinyl alcohol, hydroxypropylcellulose, hydroxypropylmethylcellulose and combinations thereof;
at least one disintegrant selected from the group consisting of sodium starch glycolate, croscarmellose sodium, crospovidone and combinations thereof;
at least one lubricant selected from the group consisting of magnesium stearate, calcium stearate, talc, aluminum monostearate, aluminum distearate, aluminum tristearate, zinc stearate, stearic acid, sodium stearyl fumarate, magnesium behenate, calcium behenate, magnesium arachinate, calcium arachinate, and combinations thereof;
at least one filler which comprises microcrystalline cellulose in an amount of 10 to 30% by weight, relative to the weight of the pharmaceutical composition, and at least one further compound selected from the group consisting of mannitol, calcium hydrogen phosphate, lactose and starch.

In a further preferred embodiment, the pharmaceutical composition of the present invention comprises
lapatinib or a pharmaceutically acceptable salt or solvate or solvated salt thereof,
at least one binder selected from the group consisting of gelatine, polyvinyl pyrrolidone, polyvinyl acetate, polyvinyl alcohol, hydroxypropylcellulose, hydroxypropylmethylcellulose and combinations thereof;
at least one disintegrant selected from the group consisting of sodium starch glycolate, croscarmellose sodium, crospovidone and combinations thereof;
at least one lubricant selected from the group consisting of magnesium stearate, calcium stearate, talc, aluminum monostearate, aluminum distearate, aluminum tristearate, zinc stearate, stearic acid, sodium stearyl fumarate, magnesium behenate, calcium behenate, magnesium arachinate, calcium arachinate, and combinations thereof;
at least one filler which comprises microcrystalline cellulose in an amount of 10 to 30% by weight, relative to the weight of the pharmaceutical composition, and 10 to 20% by weight, relative to the weight of the pharmaceutical composition, of at least one further compound selected from the group consisting of mannitol, calcium hydrogen phosphate, lactose and starch.

In a further preferred embodiment, the pharmaceutical composition of the present invention comprises
lapatinib or a pharmaceutically acceptable salt or solvate or solvated salt thereof,
at least one binder selected from the group consisting of gelatine, polyvinyl pyrrolidone, polyvinyl acetate, polyvinyl alcohol, hydroxypropylcellulose, hydroxypropylmethylcellulose and combinations thereof;
at least one disintegrant selected from the group consisting of sodium starch glycolate, croscarmellose sodium, crospovidone and combinations thereof;
at least one lubricant selected from the group consisting of magnesium stearate, calcium stearate, talc, aluminum monostearate, aluminum distearate, aluminum tristearate, zinc stearate, stearic acid, sodium stearyl fumarate, magnesium behenate, calcium behenate, magnesium arachinate, calcium arachinate, and combinations thereof;
at least one filler which comprises microcrystalline cellulose in an amount of 10 to 30% by weight, relative to the weight of the pharmaceutical composition, and lactose in an amount of 10 to 20% by weight, relative to the weight of the pharmaceutical composition.

In a further preferred embodiment, the pharmaceutical composition of the present invention comprises
lapatinib or a pharmaceutically acceptable salt or solvate or solvated salt thereof,
a binder selected from the group consisting of polyvinyl pyrrolidone, polyvinyl acetate, polyvinyl alcohol, hydroxypropylcellulose, hydroxypropylmethylcellulose and combinations thereof;
a disintegrant selected from the group consisting of sodium starch glycolate, croscarmellose sodium, crospovidone and combinations thereof;
a lubricant selected from the group consisting of magnesium stearate, calcium stearate, sodium stearyl fumarate, magnesium behenate, calcium behenate, magnesium arachinate, calcium arachinate and combinations thereof;
at least one filler which comprises microcrystalline cellulose in an amount of 10 to 30% by weight, relative to the weight of the pharmaceutical composition.

In a further preferred embodiment, the pharmaceutical composition of the present invention comprises
lapatinib or a pharmaceutically acceptable salt or solvate or solvated salt thereof,
a binder selected from the group consisting of polyvinyl pyrrolidone, polyvinyl acetate, polyvinyl alcohol, hydroxypropylcellulose, hydroxypropylmethylcellulose and combinations thereof;
a disintegrant selected from the group consisting of sodium starch glycolate, croscarmellose sodium, crospovidone and combinations thereof;
a lubricant selected from the group consisting of magnesium stearate, calcium stearate, sodium stearyl fumarate, magnesium behenate, calcium behenate, magnesium arachinate, calcium arachinate and combinations thereof;
at least one filler which comprises microcrystalline cellulose in an amount of 10 to 30% by weight, relative to the weight of the pharmaceutical composition, and at least one further compound selected from the group consisting of mannitol, calcium hydrogen phosphate, lactose and starch.

In a further preferred embodiment, the pharmaceutical composition of the present invention comprises
lapatinib or a pharmaceutically acceptable salt or solvate or solvated salt thereof,
a binder selected from the group consisting of polyvinyl pyrrolidone, polyvinyl acetate, polyvinyl alcohol, hydroxypropylcellulose, hydroxypropylmethylcellulose and combinations thereof;
a disintegrant selected from the group consisting of sodium starch glycolate, croscarmellose sodium, crospovidone and combinations thereof;
a lubricant selected from the group consisting of magnesium stearate, calcium stearate, sodium stearyl fumarate, magnesium behenate, calcium behenate, magnesium arachinate, calcium arachinate and combinations thereof;
at least one filler which comprises microcrystalline cellulose in an amount of 10 to 30% by weight, relative to the weight of the pharmaceutical composition, and 10 to 20% by weight, relative to the weight of the pharmaceutical composition, of at least one further compound selected from the group consisting of mannitol, calcium hydrogen phosphate, lactose and starch.

In a further preferred embodiment, the pharmaceutical composition of the present invention comprises
lapatinib or a pharmaceutically acceptable salt or solvate or solvated salt thereof,
a binder selected from the group consisting of polyvinyl pyrrolidone, polyvinyl acetate, polyvinyl alcohol, hydroxypropylcellulose, hydroxypropylmethylcellulose and combinations thereof;
a disintegrant selected from the group consisting of sodium starch glycolate, croscarmellose sodium, crospovidone and combinations thereof;
a lubricant selected from the group consisting of magnesium stearate, calcium stearate, sodium stearyl fumarate, magnesium behenate, calcium behenate, magnesium arachinate, calcium arachinate and combinations thereof;
at least one filler which comprises microcrystalline cellulose in an amount of 10 to 30% by weight, relative to the weight of the pharmaceutical composition, and lactose in an amount of 10 to 20% by weight, relative to the weight of the pharmaceutical composition.

In a further preferred embodiment, the pharmaceutical composition of the present invention comprises
lapatinib or a pharmaceutically acceptable salt or solvate or solvated salt thereof,
a binder selected from the group consisting of polyvinyl pyrrolidone, hydroxypropylmethylcellulose and combinations thereof;
a disintegrant selected from the group consisting of sodium starch glycolate, croscarmellose sodium and combinations thereof;
a lubricant selected from the group consisting of magnesium stearate, calcium stearate, sodium stearyl fumarate, magnesium behenate, calcium behenate, magnesium arachinate, calcium arachinate and combinations thereof;
a filler which comprises microcrystalline cellulose in an amount of 10 to 30% by weight, relative to the weight of the pharmaceutical composition, and 10 to 20% by weight, relative to the weight of the pharmaceutical composition, of at least one further compound selected from the group consisting of mannitol, calcium hydrogen phosphate, lactose and starch.

In a further preferred embodiment, the pharmaceutical composition of the present invention comprises
lapatinib ditosylate monohydrate,
a binder selected from the group consisting of polyvinyl pyrrolidone, hydroxypropylmethylcellulose and combinations thereof;
a disintegrant selected from the group consisting of sodium starch glycolate, croscarmellose sodium and combinations thereof;
a lubricant selected from the group consisting of magnesium stearate, calcium stearate, sodium stearyl fumarate, magnesium behenate, calcium behenate, magnesium arachinate, calcium arachinate and combinations thereof;
a filler which comprises microcrystalline cellulose in an amount of 10 to 30% by weight, relative to the weight of the pharmaceutical composition, and 10 to 20% by weight, relative to the weight of the pharmaceutical composition, of lactose.

In a further preferred embodiment, the pharmaceutical composition of the present invention comprises
30 to 60% by weight, relative to the weight of the pharmaceutical composition, of lapatinib or a pharmaceutically acceptable salt or solvate or solvated salt thereof,
1 to 20% by weight, relative to the weight of the pharmaceutical composition, of at least one binder selected from the group consisting of gelatine, polyvinyl pyrrolidone, polyvinyl acetate, polyvinyl alcohol, hydroxypropylcellulose, hydroxypropylmethylcellulose and combinations thereof;
0.5 to 8% by weight, relative to the weight of the pharmaceutical composition, of at least one disintegrant selected from the group consisting of sodium starch glycolate, croscarmellose sodium, crospovidone and combinations thereof;
0.01 to 3% by weight, relative to the weight of the pharmaceutical composition, of at least one lubricant selected from the group consisting of magnesium stearate, calcium stearate, talc, aluminum monostearate, aluminum distearate, aluminum tristearate, zinc stearate, stearic acid, sodium stearyl fumarate, magnesium behenate, calcium behenate, magnesium arachinate, calcium arachinate, and combinations thereof;
at least one filler which comprises microcrystalline cellulose in an amount of 10 to 30% by weight, relative to the weight of the pharmaceutical composition.

In a further preferred embodiment, the pharmaceutical composition of the present invention comprises
30 to 60% by weight, relative to the weight of the pharmaceutical composition, of lapatinib or a pharmaceutically acceptable salt or solvate or solvated salt thereof,
1 to 20% by weight, relative to the weight of the pharmaceutical composition, of at least one binder selected from the group consisting of gelatine, polyvinyl pyrrolidone, polyvinyl acetate, polyvinyl alcohol, hydroxypropylcellulose, hydroxypropylmethylcellulose and combinations thereof;
0.5 to 8% by weight, relative to the weight of the pharmaceutical composition, of at least one disintegrant selected from the group consisting of sodium starch glycolate, croscarmellose sodium, crospovidone and combinations thereof;
0.01 to 3% by weight, relative to the weight of the pharmaceutical composition, of at least one lubricant selected from the group consisting of magnesium stearate, calcium stearate, talc, aluminum monostearate, aluminum distearate, aluminum tristearate, zinc stearate, stearic acid, sodium stearyl fumarate, magnesium behenate, calcium behenate, magnesium arachinate, calcium arachinate, and combinations thereof;
at least one filler which comprises microcrystalline cellulose in an amount of 10 to 30% by weight, relative to the weight of the pharmaceutical composition, and 10 to 20% by weight, relative to the weight of the pharmaceutical composition, of at least one further compound selected from the group consisting of mannitol, calcium hydrogen phosphate, lactose and starch.

In a further preferred embodiment, the pharmaceutical composition of the present invention comprises
30 to 60% by weight, relative to the weight of the pharmaceutical composition, of lapatinib or a pharmaceutically acceptable salt or solvate or solvated salt thereof,
1 to 20% by weight, relative to the weight of the pharmaceutical composition, of at least one binder selected from the group consisting of gelatine, polyvinyl pyrrolidone, polyvinyl acetate, polyvinyl alcohol, hydroxypropylcellulose, hydroxypropylmethylcellulose and combinations thereof;
0.5 to 8% by weight, relative to the weight of the pharmaceutical composition, of at least one disintegrant selected from the group consisting of sodium starch glycolate, croscarmellose sodium, crospovidone and combinations thereof;
0.01 to 3% by weight, relative to the weight of the pharmaceutical composition, of at least one lubricant selected from the group consisting of magnesium stearate, calcium stearate, talc, aluminum monostearate, aluminum distearate, aluminum tristearate, zinc stearate, stearic acid, sodium stearyl fumarate, magnesium behenate, calcium behenate, magnesium arachinate, calcium arachinate, and combinations thereof;
at least one filler which comprises microcrystalline cellulose in an amount of 10 to 30% by weight, relative to the weight of the pharmaceutical composition, and lactose in an amount of 10 to 20% by weight, relative to the weight of the pharmaceutical composition.

In a further preferred embodiment, the pharmaceutical composition of the present invention comprises
30 to 60% by weight, relative to the weight of the pharmaceutical composition, of lapatinib or a pharmaceutically acceptable salt or solvate or solvated salt thereof,
1 to 20% by weight, relative to the weight of the pharmaceutical composition, of a binder selected from the group consisting of polyvinyl pyrrolidone, polyvinyl acetate, polyvinyl alcohol, hydroxypropylcellulose, hydroxypropylmethylcellulose and combinations thereof;
0.5 to 8% by weight, relative to the weight of the pharmaceutical composition, of a disintegrant selected from the group consisting of sodium starch glycolate, croscarmellose sodium, crospovidone and combinations thereof;
0.01 to 3% by weight, relative to the weight of the pharmaceutical composition, of a lubricant selected from the group consisting of magnesium stearate, calcium stearate, sodium stearyl fumarate, magnesium behenate, calcium behenate, magnesium arachinate, calcium arachinate and combinations thereof;
at least one filler which comprises microcrystalline cellulose in an amount of 10 to 30% by weight, relative to the weight of the pharmaceutical composition.

In a further preferred embodiment, the pharmaceutical composition of the present invention comprises
30 to 60% by weight, relative to the weight of the pharmaceutical composition, of lapatinib or a pharmaceutically acceptable salt or solvate or solvated salt thereof,
1 to 20% by weight, relative to the weight of the pharmaceutical composition, of a binder selected from the group consisting of polyvinyl pyrrolidone, polyvinyl acetate, polyvinyl alcohol, hydroxypropylcellulose, hydroxypropylmethylcellulose and combinations thereof;
0.5 to 8% by weight, relative to the weight of the pharmaceutical composition, of a disintegrant selected from the group consisting of sodium starch glycolate, croscarmellose sodium, crospovidone and combinations thereof;
0.01 to 3% by weight, relative to the weight of the pharmaceutical composition, of a lubricant selected from the group consisting of magnesium stearate, calcium stearate, sodium stearyl fumarate, magnesium behenate, calcium behenate, magnesium arachinate, calcium arachinate and combinations thereof;
at least one filler which comprises microcrystalline cellulose in an amount of 10 to 30% by weight, relative to the weight of the pharmaceutical composition, and 10 to 20% by weight, relative to the weight of the pharmaceutical composition, of at least one further compound selected from the group consisting of mannitol, calcium hydrogen phosphate, lactose and starch.

In a further preferred embodiment, the pharmaceutical composition of the present invention comprises
30 to 60% by weight, relative to the weight of the pharmaceutical composition, of lapatinib or a pharmaceutically acceptable salt or solvate or solvated salt thereof,
1 to 20% by weight, relative to the weight of the pharmaceutical composition, of a binder selected from the group consisting of polyvinyl pyrrolidone, polyvinyl acetate, polyvinyl alcohol, hydroxypropylcellulose, hydroxypropylmethylcellulose and combinations thereof; 0.5 to 8% by weight, relative to the weight of the pharmaceutical composition, of a disintegrant selected from the group consisting of sodium starch glycolate, croscarmellose sodium, crospovidone and combinations thereof;
0.01 to 3% by weight, relative to the weight of the pharmaceutical composition, of a lubricant selected from the group consisting of magnesium stearate, calcium stearate, sodium stearyl fumarate, magnesium behenate, calcium behenate, magnesium arachinate, calcium arachinate and combinations thereof;
at least one filler which comprises microcrystalline cellulose in an amount of 10 to 30% by weight, relative to the weight of the pharmaceutical composition, and lactose in an amount of 10 to 20% by weight, relative to the weight of the pharmaceutical composition.

In a further preferred embodiment, the pharmaceutical composition of the present invention comprises
30 to 60% by weight, relative to the weight of the pharmaceutical composition, of lapatinib or a pharmaceutically acceptable salt or solvate or solvated salt thereof,
1 to 20% by weight, relative to the weight of the pharmaceutical composition, of a binder selected from the group consisting of polyvinyl pyrrolidone, hydroxypropylmethylcellulose and combinations thereof;
0.5 to 8% by weight, relative to the weight of the pharmaceutical composition, of a disintegrant selected from the group consisting of sodium starch glycolate, croscarmellose sodium and combinations thereof;
0.01 to 3% by weight, relative to the weight of the pharmaceutical composition, of a lubricant selected from the group consisting of magnesium stearate, calcium stearate, sodium stearyl fumarate, magnesium behenate, calcium behenate, magnesium arachinate, calcium arachinate and combinations thereof;
a filler which comprises microcrystalline cellulose in an amount of 10 to 30% by weight, relative to the weight of the pharmaceutical composition, and 10 to 20% by weight, relative to the weight of the pharmaceutical composition, of lactose.

In a further preferred embodiment, the pharmaceutical composition of the present invention comprises
40 to 60% by weight, relative to the weight of the pharmaceutical composition, of lapatinib ditosylate monohydrate,
5 to 10% by weight, relative to the weight of the pharmaceutical composition, of a binder selected from the group consisting of polyvinyl pyrrolidone, hydroxypropylmethylcellulose and combinations thereof;
4 to 6% by weight, relative to the weight of the pharmaceutical composition, of a disintegrant selected from the group consisting of sodium starch glycolate, croscarmellose sodium and combinations thereof;
0.5 to 2% by weight, relative to the weight of the pharmaceutical composition, of a lubricant selected from the group consisting of magnesium stearate, calcium stearate, sodium stearyl fumarate, magnesium behenate, calcium behenate, magnesium arachinate, calcium arachinate and combinations thereof;
a filler which comprises microcrystalline cellulose in an amount of 10 to 30% by weight, relative to the weight of the pharmaceutical composition, and 10 to 20% by weight, relative to the weight of the pharmaceutical composition, of lactose.

The pharmaceutical composition of the present invention can further comprise a glidant, for example, colloidal silicon dioxide.

The pharmaceutical composition of the present invention can further comprise a colorant, for example, ferric oxide.

The pharmaceutical composition according to the present invention is preferably a tablet or coated tablet. More preferably, the pharmaceutical composition according to the present invention is a coated tablet.

The coating may comprise for example a cellulose, such as hydroxypropylmethylcellulose, a polyethylene glycol, such as Macrogol 400, a sorbitan fatty acid ester, such as Polysorbate 80, and/or titanium dioxide. Optionally, the coating may comprise a colorant, such as ferric oxide or an aluminum lake (e.g. sunset yellow aluminum lake E110). Commercially available coating compositions, such as Opadry coating compositions (e.g. Opadry® YS-1-13065-A) can be used.

The pharmaceutical composition according to the present invention can be prepared by a process comprising a wet granulation step. Examples of wet granulation include wet granulation in a swaying granulator, high shear wet granulation, and fluidized bed granulation. High shear wet granulation is preferred.

The pharmaceutical composition according to the present invention can be produced by a process comprising:
(a) granulating a mixture comprising lapatinib or a pharmaceutically acceptable salt or solvate or solvated salt thereof, a filler, and optionally a disintegrant, using an aqueous solution of the binder as granulation liquid;
(b) drying the granules obtained in step (a);
(c) mixing the dried granules with the lubricant, and optionally the disintegrant;
provided that the disintegrant is added in step (a) and/or (c).

If the pharmaceutical composition according to the present invention is a tablet or coated tablet, the process may comprise an additional step of compressing the mixture obtained in step (c) into tablets.

Preferably, the granules are dried to a water content of not more than 3% by weight, more preferably of from 0.5 to 3% by weight, in above step (b). The water content can be determined as described in Ph. Eur. 7.0 by measuring the loss on drying (determined on 1 g of the granules dried in an oven at 105°C for 3 h).

A preferred process for preparing the pharmaceutical composition according to the present invention includes the following steps:
A dry blend of lapatinib or the pharmaceutically acceptable salt or solvate or solvated salt thereof, the filler and a part of the disintegrant (optionally all sieved before through a Frewitt 1.0 mm sieve) is filled into a wet granulator, such as a Glatt VG 25 granulator. The binder is dissolved in a suitable amount of purified water and then added to the dry blend under continuous stirring. Wet granulation is preferably performed at a temperature of from 20 to 30°C. The wet granules are then dried in a fluidized bed dryer, such as a Glatt GPCG 3/5 dryer, preferably at a temperature of from 30 to 40°C. The granules are sieved, e.g. through a Frewitt 1.0 mm sieve, and blended for 5 to 10 minutes in a turbula mixer at 25 rpm with the remaining part of the disintegrant. The lubricant, preferably after having been sieved through a 0.5 mm sieve, is added to the resulting blend, and the final blend is mixed in a turbula mixer at 25 rpm for 1 to 5 minutes. The final blend is compressed into tablets, for example in a Kilian RTS 24 tablet press. Optionally, the tablets are coated, for example in a Glatt Mini coater (0.8 I drum) using a suitable film-coating preparation, for example Opadry® YS-1-13065-A.

Another preferred process for preparing the pharmaceutical composition according to the present invention includes the following steps:
A dry blend of lapatinib or the pharmaceutically acceptable salt or solvate or solvated salt thereof, the filler and a part of the disintegrant (optionally all sieved before through a Frewitt 1.0 mm sieve) is filled into a fluid-bed-granulator, such as a Glatt GPCG 3/5 granulator. The binder is dissolved in a suitable amount of purified water. Fluid-bed-granulation is started and the binder solution sprayed on top of the dry blend (e.g. through a 1.0 mm spraying nozzle) preferably at a temperature of from 20 to 30°C. The wet granules are then dried in the fluid-bed-granulator preferably at a temperature of from 30 to 40°C. The granules are sieved, e.g. through a Frewitt 1.0 mm sieve, and blended for 5 to 10 minutes in a turbula mixer at 25 rpm with the remaining part of the disintegrant (optionally sieved before through a Frewitt 1.0 mm sieve). The lubricant, preferably after having been sieved through a 0.5 mm sieve, is added to the resulting blend and the final blend is mixed in a turbula mixer at 25 rpm for 1 to 5 minutes. The final blend is compressed into tablets, for example in a Kilian RTS 24 tablet press. Optionally, the tablets are coated, for example in a Glatt Mini coater (0.8 I drum) using a suitable film-coating preparation, for example Opadry® YS-1-13065-A.

The pharmaceutical composition according to the present invention optionally comprises a coating. The coating can be prepared by
(i) suspending the components of the coating in water, an organic solvent (for example ethanol) or a mixture of water and an organic solvent (for example a water/ethanol mixture),
(ii) coating the pharmaceutical composition with the thus obtained suspension using a suitable coating device, for example a fluid-bed-coater or a pan coater, and
(iii) drying the coated pharmaceutical composition.

The pharmaceutical composition of the present invention may be useful in the treatment of cancer including breast cancer, ovarian cancer, brain cancer, head cancer, neck cancer, gastric cancer, colorectal cancer, prostate cancer, bladder cancer or prostate cancer. The pharmaceutical composition of the present invention may further be useful in the treatment of psoriasis.

The pharmaceutical composition of the present invention can comprise a further active ingredient selected from anti-neoplastic agents, such as paclitaxel, docetaxel, gemcitabine, irinotecan, topotecan, cisplatin, carboplatin, oxaliplatin, capecitabine, anastrozole, letrozole, pazopanib, imatinib, erlotinib, dasatinib, sorafenib, sunitinib and gefitinib.

### EXAMPLES

### Example 1

| Component | Content in film-coated tablet [% by weight] |
|---|---|
| Lapatinib ditosylate monohydrate | 60.0 |
| Polyvinyl pyrrolidone | 7.5 |
| Sodium starch glycolate | 5.5 |
| Microcrystalline cellulose | 22.6 |
| Magnesium stearate | 1.4 |
| Film coating | 3.0 |

Total weight of film-coated tablet: 675 mg

Each of the lapatinib ditosylate monohydrate, microcrystalline cellulose and sodium starch glycolate are sieved through a Frewitt 1.0 mm sieve. A dry blend of the sieved lapatinib ditosylate monohydrate, the sieved microcrystalline cellulose and half of the sieved sodium starch glycolate is filled into a wet granulator (Glatt, VG 25 granulator). The polyvinyl pyrrolidone is dissolved in purified water (5 parts per weight of water per 1 part per weight of polyvinyl pyrrolidone) and then added to the dry blend under continuous stirring. Wet granulation is performed at 20°C. The wet granules are then dried in a fluidized bed dryer (Glatt, GPCG 3/5) at 35°C. The granules are sieved through a Frewitt 1.0 mm sieve and blended for 10 minutes in a turbula mixer at 25 rpm with the second half of the sieved sodium starch glycolate. Magnesium stearate is added to the resulting blend after having been sieved through a 0.5 mm hand sieve and the blend thus obtained is mixed in a turbula mixer at 25 rpm for 3 minutes. The final blend is compressed into tablets using a Kilian RTS 24 tablet press. The tablets are coated in a Glatt Mini coater (0.8 I drum) using a the commercially available Opadry® YS-1-13065-A film coating.

### Example 2

| Component | Content in film-coated tablet [% by weight] |
|---|---|
| Lapatinib ditosylate monohydrate | 60.0 |
| Polyvinyl pyrrolidone | 7.5 |
| Sodium starch glycolate | 5.5 |
| Microcrystalline cellulose | 22.6 |
| Calcium behenate | 1.4 |
| Film coating | 3.0 |

Total weight of film-coated tablet: 675 mg

The coated tablets are prepared as described in Example 1 with the exception that calcium behenate is used in place of magnesium stearate.

### Example 3

| Component | Content in film-coated tablet [% by weight] |
|---|---|
| Lapatinib ditosylate monohydrate | 60.0 |
| Polyvinyl pyrrolidone | 7.5 |
| Sodium starch glycolate | 5.5 |
| Microcrystalline cellulose | 22.6 |
| Calcium stearate | 1.4 |
| Film coating | 3.0 |

Total weight of film-coated tablet: 675 mg

The coated tablets are prepared as described in Example 1 with the exception that calcium stearate is used in place of magnesium stearate.

### Example 4

| Component | Content in film-coated tablet [% by weight] |
|---|---|
| Lapatinib ditosylate monohydrate | 60.0 |
| HPMC | 7.5 |
| Sodium starch glycolate | 5.5 |
| Microcrystalline cellulose | 22.6 |
| Magnesium stearate | 1.4 |
| Film coating | 3.0 |

Total weight of film-coated tablet: 675 mg

The coated tablets are prepared as described in Example 1 with the exception that HPMC is used in place of polyvinyl pyrrolidone.

### Example 5

| Component | Content in film-coated tablet [% by weight] |
|---|---|
| Lapatinib ditosylate monohydrate | 60.0 |
| Polyvinyl pyrrolidone | 7.5 |
| Croscarmellose sodium | 5.5 |
| Microcrystalline cellulose | 22.6 |
| Magnesium stearate | 1.4 |
| Film coating | 3.0 |

Total weight of film-coated tablet: 675 mg

The coated tablets are prepared as described in Example 1 with the exception that croscarmellose sodium is used in place of sodium starch glycolate.

### Example 6

| Component | Content in film-coated tablet [% by weight] |
|---|---|
| Lapatinib ditosylate monohydrate | 60.0 |
| HPMC | 7.5 |
| Croscarmellose sodium | 5.5 |
| Microcrystalline cellulose | 22.6 |
| Magnesium stearate | 1.4 |
| Film coating | 3.0 |

Total weight of film-coated tablet: 675 mg

The coated tablets are prepared as described in Example 1 with the exception that HPMC is used in place of polyvinyl pyrrolidone and croscarmellose sodium is used in place of sodium starch glycolate.

### Example 7

| Component | Content in film-coated tablet [% by weight] |
|---|---|
| Lapatinib ditosylate monohydrate | 60.0 |
| Polyvinyl pyrrolidone | 7.5 |
| Sodium starch glycolate | 5.5 |
| Microcrystalline cellulose | 12.6 |
| Alpha-lactose monohydrate | 10.0 |
| Magnesium stearate | 1.4 |
| Film coating | 3.0 |

Total weight of film-coated tablet: 675 mg

Each of the lapatinib ditosylate monohydrate, microcrystalline cellulose, alpha-lactose monohydrate and sodium starch glycolate are sieved through a Frewitt 1.0 mm sieve. A dry blend of the sieved lapatinib ditosylate monohydrate, the sieved microcrystalline cellulose, the sieved alpha-lactose monohydrate and half of the sieved sodium starch glycolate is filled into a wet granulator (Glatt, VG 25 granulator). The polyvinyl pyrrolidone is dissolved in purified water (5 parts per weight of water per 1 part per weight of polyvinyl pyrrolidone) and then added to the dry blend under continuous stirring. Wet granulation is performed at 20°C. The wet granules are then dried in a fluidized bed dryer (Glatt, GPCG 3/5) at 35°C. The granules are sieved through a Frewitt 1.0 mm sieve and blended for 10 minutes in a turbula mixer at 25 rpm with the second half of the sieved sodium starch glycolate. Magnesium stearate is added to the resulting blend after having been sieved through a 0.5 mm hand sieve and the blend thus obtained is mixed in a turbula mixer at 25 rpm for 3 minutes. The final blend is compressed into tablets using a Kilian RTS 24 tablet press. The tablets are coated in a Glatt Mini coater (0.8 I drum) using a commercially available Opadry® YS-1-13065-A film coating.

### Example 8

| Component | Content in film-coated tablet [% by weight] |
|---|---|
| Lapatinib ditosylate monohydrate | 60.0 |
| HPMC | 7.5 |
| Sodium starch glycolate | 5.5 |
| Microcrystalline cellulose | 12.6 |
| Alpha-lactose monohydrate | 10.0 |
| Magnesium stearate | 1.4 |
| Film coating | 3.0 |

Total weight of film-coated tablet: 675 mg

The coated tablets are prepared as described in Example 7 with the exception that HPMC is used in place of polyvinyl pyrrolidone.

### Example 9

| Component | Content in film-coated tablet [% by weight] |
|---|---|
| Lapatinib ditosylate monohydrate | 60.0 |
| Polyvinyl pyrrolidone | 7.5 |
| Croscarmellose sodium | 5.5 |
| Microcrystalline cellulose | 12.6 |
| Alpha-lactose monohydrate | 10.0 |
| Magnesium stearate | 1.4 |
| Film coating | 3.0 |

Total weight of film-coated tablet: 675 mg

The coated tablets are prepared as described in Example 7 with the exception that croscarmellose sodium is used in place of sodium starch glycolate.

### Example 10

| Component | Content in film-coated tablet [% by weight] |
|---|---|
| Lapatinib ditosylate monohydrate | 60.0 |
| HPMC | 7.5 |
| Croscarmellose sodium | 5.5 |
| Microcrystalline cellulose | 12.6 |
| Alpha-lactose monohydrate | 10.0 |
| Magnesium stearate | 1.4 |
| Film coating | 3.0 |

Total weight of film-coated tablet: 675 mg

The coated tablets are prepared as described in Example 7 with the exception that HPMC is used in place of polyvinyl pyrrolidone and croscarmellose sodium is used in place of sodium starch glycolate.

### Example 11

| Component | Content in film-coated tablet [% by weight] |
|---|---|
| Lapatinib ditosylate monohydrate | 60.0 |
| HPMC | 7.5 |
| Croscarmellose sodium | 5.5 |
| Microcrystalline cellulose | 12.6 |
| Alpha-lactose monohydrate | 10.0 |
| Calcium behenate | 1.4 |
| Film coating | 3.0 |

Total weight of film-coated tablet: 675 mg

The coated tablets are prepared as described in Example 7 with the exception that HPMC is used in place of polyvinyl pyrrolidone, croscarmellose sodium is used in place of sodium starch glycolate, and calcium behenate is used in place of magnesium stearate.

### Example 12

| Component | Content in film-coated tablet [% by weight] |
|---|---|
| Lapatinib ditosylate monohydrate | 52.0 |
| Polyvinyl pyrrolidone | 7.0 |
| Sodium starch glycolate | 5.0 |
| Microcrystalline cellulose | 18.8 |
| Alpha-lactose monohydrate | 13.0 |
| Magnesium stearate | 1.2 |
| Film coating | 3.0 |

Total weight of film-coated tablet: 779 mg

Each of the lapatinib ditosylate monohydrate, microcrystalline cellulose, alpha-lactose monohydrate and sodium starch glycolate are sieved through a Frewitt 1.0 mm sieve. A dry blend of the sieved lapatinib ditosylate monohydrate, the sieved microcrystalline cellulose, the sieved alpha-lactose monohydrate and half of the sieved sodium starch glycolate is filled into a fluid-bed-granulator (Glatt, GPCG 3/5 granulator). The polyvinyl pyrrolidone is dissolved in purified water (5 parts per weight of water per 1 part per weight of polyvinyl pyrrolidone). Fluid-bed-granulation is started and the polyvinyl pyrrolidone solution is sprayed on top of the dry blend through a 1.0 mm spraying nozzle at 25°C. The wet granules are then further dried in the fluid bed granulator at 35°C. The granules are sieved through a Frewitt 1.0 mm sieve and blended for 10 minutes in a turbula mixer at 25 rpm with the second half of the sieved sodium starch glycolate. Magnesium stearate is added to the resulting blend after having been sieved through a 0.5 mm hand sieve and the blend thus obtained is mixed in a turbula mixer at 25 rpm for 3 minutes. The final blend is compressed into tablets using a Kilian RTS 24 tablet press. The tablets are coated in a Glatt Mini coater (0.8 I drum) using a commercially available Opadry® YS-1-13065-A film coating.

### Example 13

| Component | Content in film-coated tablet [% by weight] |
|---|---|
| Lapatinib ditosylate monohydrate | 52.0 |
| Polyvinyl pyrrolidone | 7.0 |
| Sodium starch glycolate | 5.0 |
| Microcrystalline cellulose | 18.8 |
| Alpha-lactose monohydrate | 13.0 |
| Calcium stearate | 1.2 |
| Film coating | 3.0 |

Total weight of film-coated tablet: 779 mg

The coated tablets are prepared as described in Example 12 with the exception that calcium stearate is used in place of magnesium stearate.

### Example 14

| Component | Content in film-coated tablet [% by weight] |
|---|---|
| Lapatinib ditosylate monohydrate | 52.0 |
| HPMC | 7.0 |
| Sodium starch glycolate | 5.0 |
| Microcrystalline cellulose | 18.8 |
| Alpha-lactose monohydrate | 13.0 |
| Magnesium stearate | 1.2 |
| Film coating | 3.0 |

Total weight of film-coated tablet: 779 mg

The coated tablets are prepared as described in Example 12 with the exception that HPMC is used in place of polyvinyl pyrrolidone.

### Example 15

| Component | Content in film-coated tablet [% by weight] |
|---|---|
| Lapatinib ditosylate monohydrate | 52.0 |
| Polyvinyl pyrrolidone | 7.0 |
| Croscarmellose sodium | 5.0 |
| Microcrystalline cellulose | 18.8 |
| Alpha-lactose monohydrate | 13.0 |
| Magnesium stearate | 1.2 |
| Film coating | 3.0 |

Total weight of film-coated tablet: 779 mg

The coated tablets are prepared as described in Example 12 with the exception that croscarmellose sodium is used in place of sodium starch glycolate.

### Example 16

| Component | Content in film-coated tablet [% by weight] |
|---|---|
| Lapatinib ditosylate monohydrate | 52.0 |
| HPMC | 7.0 |
| Croscarmellose sodium | 5.0 |
| Microcrystalline cellulose | 18.8 |
| Alpha-lactose monohydrate | 13.0 |
| Magnesium stearate | 1.2 |
| Film coating | 3.0 |

Total weight of film-coated tablet: 779 mg

The coated tablets are prepared as described in Example 12 with the exception that HPMC is used in place of polyvinyl pyrrolidone and croscarmellose sodium is used in place of sodium starch glycolate.

### Example 17

| Component | Content in film-coated tablet [% by weight] |
|---|---|
| Lapatinib ditosylate monohydrate | 52.0 |
| HPMC | 7.0 |
| Croscarmellose sodium | 5.0 |
| Microcrystalline cellulose | 18.8 |
| Alpha-lactose monohydrate | 13.0 |
| Calcium stearate | 1.2 |
| Film coating | 3.0 |

Total weight of film-coated tablet: 779 mg

The coated tablets are prepared as described in Example 12 with the exception that HPMC is used in place of polyvinyl pyrrolidone, croscarmellose sodium is used in place of sodium starch glycolate, and calcium stearate is used in place of magnesium stearate.

### Example 18

| Component | Content in film-coated tablet [% by weight] |
|---|---|
| Lapatinib ditosylate monohydrate | 44.0 |
| Polyvinyl pyrrolidone | 6.5 |
| Sodium starch glycolate | 4.5 |
| Microcrystalline cellulose | 25.0 |
| Alpha-lactose monohydrate | 16.0 |
| Magnesium stearate | 1.0 |
| Film coating | 3.0 |

Total weight of film-coated tablet: 920 mg

The coated tablets are prepared as described in Example 7 with the exception that the components are used in the amounts as indicated in the above table.

### Example 19

| Component | Content in film-coated tablet [% by weight] |
|---|---|
| Lapatinib ditosylate monohydrate | 44.0 |
| Polyvinyl pyrrolidone | 6.5 |
| Sodium starch glycolate | 4.5 |
| Microcrystalline cellulose | 25.0 |
| Alpha-lactose monohydrate | 16.0 |
| Calcium arachinate | 1.0 |
| Film coating | 3.0 |

Total weight of film-coated tablet: 920 mg

The coated tablets are prepared as described in Example 18 with the exception that calcium arachinate is used in place of magnesium stearate.

### Example 20

| Component | Content in film-coated tablet [% by weight] |
|---|---|
| Lapatinib ditosylate monohydrate | 44.0 |
| HPMC | 6.5 |
| Sodium starch glycolate | 4.5 |
| Microcrystalline cellulose | 25.0 |
| Alpha-lactose monohydrate | 16.0 |
| Magnesium stearate | 1.0 |
| Film coating | 3.0 |

Total weight of film-coated tablet: 920 mg

The coated tablets are prepared as described in Example 18 with the exception that HPMC is used in place of polyvinyl pyrrolidone.

### Example 21

| Component | Content in film-coated tablet [% by weight] |
|---|---|
| Lapatinib ditosylate monohydrate | 44.0 |
| Polyvinyl pyrrolidone | 6.5 |
| Croscarmellose sodium | 4.5 |
| Microcrystalline cellulose | 25.0 |
| Alpha-lactose monohydrate | 16.0 |
| Magnesium stearate | 1.0 |
| Film coating | 3.0 |

Total weight of film-coated tablet: 920 mg

The coated tablets are prepared as described in Example 18 with the exception that croscarmellose sodium is used in place of sodium starch glycolate.

### Example 22

| Component | Content in film-coated tablet [% by weight] |
|---|---|
| Lapatinib ditosylate monohydrate | 44.0 |
| HPMC | 6.5 |
| Croscarmellose sodium | 4.5 |
| Microcrystalline cellulose | 25.0 |
| Alpha-lactose monohydrate | 16.0 |
| Magnesium stearate | 1.0 |
| Film coating | 3.0 |

Total weight of film-coated tablet: 920 mg

The coated tablets are prepared as described in Example 18 with the exception that HPMC is used in place of polyvinyl pyrrolidone and croscarmellose sodium is used in place of sodium starch glycolate.

### Example 23

| Component | Content in film-coated tablet [% by weight] |
|---|---|
| Lapatinib ditosylate monohydrate | 44.0 |
| HPMC | 6.5 |
| Croscarmellose sodium | 4.5 |
| Microcrystalline cellulose | 25.0 |
| Alpha-lactose monohydrate | 16.0 |
| Calcium arachinate | 1.0 |
| Film coating | 3.0 |

Total weight of film-coated tablet: 920 mg

The coated tablets are prepared as described in Example 18 with the exception that HPMC is used in place of polyvinyl pyrrolidone, croscarmellose sodium is used in place of sodium starch glycolate, and calcium arachinate is used in place of magnesium stearate.

### Example 24

| Component | Content in film-coated tablet [% by weight] |
|---|---|
| Lapatinib ditosylate monohydrate | 40.5 |
| Polyvinyl pyrrolidone | 6.2 |
| Sodium starch glycolate | 4.3 |
| Microcrystalline cellulose | 28.0 |
| Alpha-lactose monohydrate | 17.0 |
| Magnesium stearate | 1.0 |
| Film coating | 3.0 |

Total weight of film-coated tablet: 1000 mg

The coated tablets are prepared as described in Example 12 with the exception that the components are used in the amounts as indicated in the above table.

### Example 25

| Component | Content in film-coated tablet [% by weight] |
|---|---|
| Lapatinib ditosylate monohydrate | 40.5 |
| Polyvinyl pyrrolidone | 6.2 |
| Sodium starch glycolate | 4.3 |
| Microcrystalline cellulose | 28.0 |
| Alpha-lactose monohydrate | 17.0 |
| Calcium stearate | 1.0 |
| Film coating | 3.0 |

Total weight of film-coated tablet: 1000 mg

The coated tablets are prepared as described in Example 24 with the exception that calcium stearate is used in place of magnesium stearate.

### Example 26

| Component | Content in film-coated tablet [% by weight] |
|---|---|
| Lapatinib ditosylate monohydrate | 40.5 |
| HPMC | 6.2 |
| Sodium starch glycolate | 4.3 |
| Microcrystalline cellulose | 28.0 |
| Alpha-lactose monohydrate | 17.0 |
| Magnesium stearate | 1.0 |
| Film coating | 3.0 |

Total weight of film-coated tablet: 1000 mg

The coated tablets are prepared as described in Example 24 with the exception that HPMC is used in place of polyvinyl pyrrolidone.

### Example 27

| Component | Content in film-coated tablet [% by weight] |
|---|---|
| Lapatinib ditosylate monohydrate | 40.5 |
| Polyvinyl pyrrolidone | 6.2 |
| Croscarmellose sodium | 4.3 |
| Microcrystalline cellulose | 28.0 |
| Alpha-lactose monohydrate | 17.0 |
| Magnesium stearate | 1.0 |
| Film coating | 3.0 |

Total weight of film-coated tablet: 1000 mg

The coated tablets are prepared as described in Example 24 with the exception that croscarmellose sodium is used in place of sodium starch glycolate.

### Example 28

| Component | Content in film-coated tablet [% by weight] |
|---|---|
| Lapatinib ditosylate monohydrate | 40.5 |
| HPMC | 6.2 |
| Croscarmellose sodium | 4.3 |
| Microcrystalline cellulose | 28.0 |
| Alpha-lactose monohydrate | 17.0 |
| Magnesium stearate | 1.0 |
| Film coating | 3.0 |

Total weight of film-coated tablet: 1000 mg

The coated tablets are prepared as described in Example 24 with the exception that HPMC is used in place of polyvinyl pyrrolidone and croscarmellose sodium is used in place of sodium starch glycolate.

### Example 29

| Component | Content in film-coated tablet [% by weight] |
|---|---|
| Lapatinib ditosylate monohydrate | 40.5 |
| HPMC | 6.2 |
| Croscarmellose sodium | 4.3 |
| Microcrystalline cellulose | 28.0 |
| Alpha-lactose monohydrate | 17.0 |
| Calcium stearate | 1.0 |
| Film coating | 3.0 |

Total weight of film-coated tablet: 1000 mg

The coated tablets are prepared as described in Example 24 with the exception that HPMC is used in place of polyvinyl pyrrolidone, croscarmellose sodium is used in place of sodium starch glycolate, and calcium stearate is used in place of magnesium stearate.

### Example 30

| Component | Content in film-coated tablet [% by weight] |
|---|---|
| Lapatinib ditosylate monohydrate | 40.5 |
| HPMC | 6.2 |
| Croscarmellose sodium | 4.3 |
| Microcrystalline cellulose | 28.0 |
| Alpha-lactose monohydrate | 17.0 |
| Sodium stearyl fumarate | 1.0 |
| Film coating | 3.0 |

Total weight of film-coated tablet: 1000 mg

The coated tablets are prepared as described in Example 24 with the exception that HPMC is used in place of polyvinyl pyrrolidone, croscarmellose sodium is used in place of sodium starch glycolate, and sodium stearyl fumarate is used in place of magnesium stearate.

## Claims

1. A pharmaceutical composition comprising an active ingredient, at least one binder, at least one disintegrant, at least one lubricant, and at least one filler, wherein the active ingredient is lapatinib or a pharmaceutically acceptable salt or solvate or solvated salt thereof; the binder is selected from the group consisting of polyvinyl pyrrolidone, hydroxypropylmethylcellulose and combinations thereof; the disintegrant is selected from the group consisting of sodium starch glycolate, croscarmellose sodium and combinations thereof; the lubricant is selected from the group consisting of magnesium stearate, calcium stearate, sodium stearyl fumarate, magnesium behenate, calcium behenate, magnesium arachinate, calcium arachinate and combinations thereof; and the filler comprises microcrystalline cellulose in an amount of 10 to 30% by weight, relative to the weight of the pharmaceutical composition, and lactose in an amount of 10 to 20% by weight, relative to the weight of the pharmaceutical composition;
and wherein the microcrystalline cellulose has a median particle size of 50 to 100µm.

2. A pharmaceutical composition according to claim 1 comprising
30 to 60% by weight, relative to the weight of the pharmaceutical composition, of lapatinib or a pharmaceutically acceptable salt or solvate or solvated salt thereof;
1 to 20% by weight, relative to the weight of the pharmaceutical composition, of a binder selected from the group consisting of polyvinyl pyrrolidone, hydroxypropylmethylcellulose and combinations thereof;
0.5 to 8% by weight, relative to the weight of the pharmaceutical composition, of a disintegrant selected from the group consisting of sodium starch glycolate, croscarmellose sodium and combinations thereof;
0.01 to 3% by weight, relative to the weight of the pharmaceutical composition, of a lubricant selected from the group consisting of magnesium stearate, calcium stearate, sodium stearyl fumarate, magnesium behenate, calcium behenate, magnesium arachinate, calcium arachinate and combinations thereof;
a filler which comprises microcrystalline cellulose in an amount of 10 to 30% by weight, relative to the weight of the pharmaceutical composition, and lactose in an amount of 10 to 20% by weight, relative to the weight of the pharmaceutical composition.

3. A pharmaceutical composition according to any of claims 1 to 2 which is a tablet or a coated tablet.

4. A pharmaceutical composition according to claim 3 which is a coated tablet, wherein the coating comprises a cellulose, a polyethylene glycol, a sorbitan fatty acid ester and/or titanium oxide.

5. Process for producing the pharmaceutical composition according to any of claims 1 to 4 comprising
(a) granulating a mixture comprising lapatinib or a pharmaceutically acceptable salt or solvate or solvated salt thereof, the filler, and optionally the disintegrant, using an aqueous solution of the binder as granulation liquid;
(b) drying the granules obtained in step (a);
(c) mixing the dried granules with the lubricant, and optionally the disintegrant; provided that the disintegrant is added in step (a) and/or (c).

6. Process according to claim 5, wherein the step (a) is a high shear wet granulation.

7. Process according to claim 5 or 6 wherein the granules are dried to a water content of 0.5 to 3% by weight.

## Patentansprüche

1. Arzneimittelzusammensetzung, die einen wirksamen Bestandteil, mindestens ein Bindemittel, mindestens ein Sprengmittel, mindestens ein Schmiermittel und mindestens einen Füllstoff aufweist, wobei der wirksame Bestandteil Lapatinib oder ein pharmazeutisch akzeptables Salz oder Solvat oder solvatisiertes Salz davon ist; das Bindemittel aus der Gruppe ausgewählt ist, die aus Polyvinylpyrrolidon, Hydroxypropylmethylcellulose und Kombinationen davon besteht; das Sprengmittel aus der Gruppe ausgewählt ist, die aus Natriumstärkeglykolat, Croscarmellose-Natrium und Kombinationen davon besteht; das Schmiermittel aus der Gruppe ausgewählt ist, die aus Magnesiumstearat, Calciumstearat, Natriumstearylfumarat, Magnesiumbehenat, Calciumbehenat, Magnesiumarachinat, Calciumarachinat und Kombinationen davon besteht; und der Füllstoff mikrokristalline Cellulose in einer Menge von 10 bis 30 Gew.-% relativ zum Gewicht der Arzneimittelzusammensetzung und Laktose in einer Menge von 10 bis 20 Gew.-% relativ zum Gewicht der Arzneimittelzusammensetzung aufweist;
und wobei die mikrokristalline Cellulose eine mittlere Teilchengröße von 50 bis 100 µm aufweist.

2. Arzneimittelzusammensetzung nach Anspruch 1, die aufweist:
30 bis 60 Gew.-% relativ zum Gewicht der Arzneimittelzusammensetzung Lapatinib oder ein pharmazeutisch akzeptables Salz oder Solvat oder solvatisiertes Salz davon;
1 bis 20 Gew.-% relativ zum Gewicht der Arzneimittelzusammensetzung eines Bindemittels, das aus der Gruppe ausgewählt ist, die aus Polyvinylpyrrolidon, Hydroxypropylmethylcellulose und Kombinationen davon besteht;
0,5 bis 8 Gew.-% relativ zum Gewicht der Arzneimittelzusammensetzung eines Sprengmittels, das aus der Gruppe ausgewählt ist, die aus Natriumstärkeglykolat, Croscarmellose-Natrium und Kombinationen davon besteht;
0,01 bis 3 Gew.-% relativ zum Gewicht der Arzneimittelzusammensetzung eines Schmiermittels, das aus der Gruppe ausgewählt ist, die aus Magnesiumstearat, Calciumstearat, Natriumstearylfumarat, Magnesiumbehenat, Calciumbehenat, Magnesiumarachinat, Calciumarachinat und Kombinationen davon besteht;
einen Füllstoff, der mikrokristalline Cellulose in einer Menge von 10 bis 30 Gew.-% relativ zum Gewicht der Arzneimittelzusammensetzung und Laktose in einer Menge von 10 bis 20 Gew.-% relativ zum Gewicht der Arzneimittelzusammensetzung aufweist.

3. Arzneimittelzusammensetzung nach einem der Ansprüche 1 bis 2, die eine Tablette oder ein Dragee ist.

4. Arzneimittelzusammensetzung nach Anspruch 3, die ein Dragee ist, wobei der Überzug Cellulose, Polyethylenglykol, Sorbitanfettsäureester und/oder Titanoxid aufweist.

5. Verfahren zum Herstellen der Arzneimittelzusammensetzung nach einem der Ansprüche 1 bis 4, das aufweist:
(a) Granulieren einer Mischung, die Lapatinib oder ein pharmazeutisch akzeptables Salz oder Solvat oder solvatisiertes Salz davon, den Füllstoff und optional das Sprengmittel aufweist, unter Verwenden einer wässrigen Lösung des Bindemittels als Granulationsflüssigkeit;
(b) Trocknen des im Schritt (a) erhaltenen Granulats;
(c) Mischen des getrockneten Granulats mit dem Schmiermittel und optional dem Sprengmittel; unter der Voraussetzung, dass das Sprengmittel im Schritt (a) und/oder (c) hinzugefügt wurde.

6. Verfahren nach Anspruch 5, wobei der Schritt (a) eine Nassgranulation mit hoher Scherung ist.

7. Verfahren nach Anspruch 5 oder 6 wobei das Granulat bis zu einem Wassergehalt von 0,5 bis 3 Gew.-% getrocknet wird.

## Revendications

1. Composition pharmaceutique comprenant un ingrédient actif, au moins un agent liant, au moins un agent désintégrant, au moins un agent lubrifiant, et au moins un agent de charge, où l'ingrédient actif est le lapatinibe ou un sel ou un solvate ou un sel solvaté pharmaceutiquement acceptable de celui-ci ; l'agent liant est sélectionné dans le groupe comprenant polyvinylpyrrolidone, hydroxypropylméthylcellulose et des combinaisons de celles-ci ; l'agent désintégrant est sélectionné dans le groupe comprenant glycolate d'amidon sodique, croscarmellose sodique et des combinaisons de ceux-ci ; l'agent lubrifiant est sélectionné dans le groupe comprenant stéarate de magnésium, stéarate de calcium, stéaryl fumarate de sodium, béhénate de magnésium, béhénate de calcium, arachinate de magnésium, arachinate de calcium et des combinaisons de ceux-ci ; et l'agent de charge comprend de la cellulose microcristalline dans une teneur comprise entre 10 et 30 % en poids, par rapport au poids de la composition pharmaceutique, et du lactose dans une teneur comprise entre 10 et 20 % en poids, par rapport au poids de la composition pharmaceutique ;
et où la cellulose microcristalline a une grandeur de particule moyenne comprise entre 50 et 100 µm.

2. Composition pharmaceutique selon la revendication 1, comprenant
de 30 à 60 % en poids, par rapport au poids de la composition pharmaceutique, de lapatinibe ou d'un sel ou solvate ou sel solvaté pharmaceutiquement acceptable de celui-ci ;
de 1 à 20 % en poids, par rapport au poids de la composition pharmaceutique, d'un agent liant sélectionné dans le groupe comprenant polyvinylpyrrolidone, hydroxypropylméthylcellulose et des combinaisons de celles-ci ;
de 0,5 à 8 % en poids, par rapport au poids de la composition pharmaceutique, d'un agent désintégrant sélectionné dans le groupe comprenant glycolate d'amidon sodique, croscarmellose sodique et des combinaisons de ceux-ci ;
de 0,01 à 3 % en poids, par rapport au poids de la composition pharmaceutique, d'un agent lubrifiant sélectionné dans le groupe comprenant stéarate de magnésium, stéarate de calcium, stéaryl fumarate de sodium, béhénate de magnésium, béhénate de calcium, arachinate de magnésium, arachinate de calcium et des combinaisons de ceux-ci ;
un agent de charge comprenant de la cellulose microcristalline dans une teneur comprise entre 10 et 30 % en poids, par rapport au poids de la composition pharmaceutique, et du lactose dans une teneur comprise entre 10 et 20 % en poids, par rapport au poids de la composition pharmaceutique.

3. Composition pharmaceutique selon la revendication 1 ou la revendication 2, laquelle est un comprimé ou un comprimé enrobé.

4. Composition pharmaceutique selon la revendication 3, laquelle est un comprimé enrobé, l'enrobage comprenant de la cellulose, du polyéthylène glycol, un ester d'acide gras de sorbitane et/ou de l'oxyde de titane.

5. Procédé de production de la composition pharmaceutique selon l'une des revendications 1 à 4, comprenant
(a) la granulation d'un mélange comprenant le lapatinibe ou un sel ou un solvate ou un sel solvaté pharmaceutiquement acceptable de celui-ci, l'agent de charge, et facultativement l'agent désintégrant, au moyen d'une solution aqueuse de l'agent liant en tant que liquide de granulation ;
(b) le séchage des granules obtenus lors de l'étape (a) ;
(c) le mélange des granules séchés avec l'agent lubrifiant, et facultativement l'agent désintégrant ; à la condition que l'agent désintégrant soit ajouté lors de l'étape (a) et/ou de l'étape (c).

6. Procédé selon la revendication 5, où l'étape (a) est une granulation humide à cisaillement élevé.

7. Procédé selon la revendication 5 ou la revendication 6, où les granules sont séchés jusqu'à obtenir une teneur d'eau comprise entre 0,5 et 3 % en poids.
